# EUROPEAN PATENT APPLICATION

(11) **EP 1 018 325 A1**
(43) Date of publication of application: **12.07.2000**
(21) Application number: 99100117.3
(22) Date of filing: 04.01.1999
(51) Int. Cl.: A61F 5/451

(54) **Container for the collection of bodily waste with visual inspection means**

(71) Applicant: THE PROCTER & GAMBLE COMPANY, Cincinnati, Ohio 45202 (US)
(72) Inventor: D'Acchioli, Vincenzo, 65779 Kelkheim am Taunus (DE); Thurnay, Susanne, 65123 Pescara (IT); Evangelista, Olindo, 66023 Francavilla al Mare, Chieti (IT)
(74) Representative: Kohol, Sonia

(57) **Abstract**

The present invention relates to a human waste management device for babies, children or adults to be attached to the perianal or uro-genital area of the wearer, said device being provided with a visual inspection means. A preferred embodiment of the present invention comprises a window (46) in the garment facing portion (17) of the bag (11) to allow for visual inspection of the inside of the bag (11).

## Description

### Field of the invention

The present invention relates to a human waste management device for babies, children or adults. Said device comprises a bag for the containment of faecal matter, said bag being designed to provide a means for visual inspection of the matter contained.

### Background of the invention

Human waste management devices are known articles of manufacture that are designed to be worn principally by incontinence sufferers and in particular by bedridden patients. Such human waste management devices are attached to the perianal or uro-genital region of the wearer and are intended to entrap and immediately contain faecal material and other bodily discharges. Such devices as they are mostly known today are constituted of a relatively long and narrow tube at one extremity of which is positioned the aperture and the attachment device, which can be adhesive.

Such bags are disclosed in e. g. the following documents: US 3,577,989, which details a disposable plastic bag for incontinence sufferers. US 4,784,656 which describes a receptacle for collecting faecal matter. The receptacle is formed from two sheets of thermoplastic film that are heat sealed along their side edges. GB 2 152 387, which teaches a faecal collector for incontinence sufferers comprising a collection bag consisting of a pair of panels of thermoplastic sheet material joined at their margins. In a preferred embodiment, the collection bag is formed from a single sheet of odour-barrier thermoplastic film folded along a vertical midline to provide a pair of continuous panels. SE 8 104 934, which discloses an oblong bag made from a thin, flexible and fluid tight material.

EP 245 064 discloses bags substantially consisting of a front and a rear wall, which are made of a synthetic plastic material, such as PVC. The front wall containing the aperture and attachment means to the body may be provided with a perforated plastic layer. GB 2 215 605 discloses an ostonomy bag comprising panels of synthetic plastic material, the rear bag wall further comprising a needled film.

Urine management devices are, for instance, disclosed in the following documents: GB 1,092,274 discloses a pediatric urine collector for female use comprising a collector bag of plastic material opening. The base of the opening is provided with a wedge like projection adapted to engage the lower perineal area of the infant. The collector is secured to the body of the wear by adhesive material, US 4,804,377 discloses a collector for urine specimens from children. The collector comprises a rectangular flange for adhesive attachment, which comprises a round, slightly oval aperture. US 1,092,274 discloses a urine collector for female infants comprising an aperture which is smaller at the bottom end than at the top end and could be described as droplet shaped. US 3,292,626 also discloses a urine collector for female infants comprising a circular or droplet shaped aperture. Chinese patent application CN 1079381 discloses urine bags for infants with circular and elliptical apertures.

All of the human waste management devices disclosed in the documents, which are referred to above, are designed for a regular change, which is in particular required due to the limited volume of the bag. Thus it is important that the wearer or carer is able to easily establish, whether or not faecal matter or urine is contained within the bag and preferably how much faecal matter or urine is contained within the bag. If no such means is provided, there is a need to detach the human waste management device and determine the content of the device via the aperture of the human waste management device.

Typically the human waste management device is adhesively attached to the skin of the wearer and such detachment and following reattachment of the same or another human waste management device is typically stressful for the skin of the wearer to which the human waste management device is attached. It is very desirable to avoid any additional stress or even pain for the skin of the wearer which may be particularly sensitive due to contact with faecal matter or urine.

Furthermore unintentional detachment of human waste management devices from the wearer is a problem known in the art (GB 2 116 849). For most designs of human waste management devices the risk of unintentional detachment increases with an increased amount of faecal matter or urine contained in the bag. Similarly rupture of the bag is a risk which increased with the amount of faecal matter or urine contained in the bag. Moreover, an indication of the filling of the bag is important for the caretaker to avoid leakage when detaching the bag.

The prior art is silent about the question of how a caretaker can establish when to change the human waste management device. The bags for human waste management devices disclosed in the prior art are mostly of a thin material which may allow some inspection, since a filled bag will typically have a different contour then an empty bag. The change in contour may be visually perceived or may be tactilely perceived when the caretaker touches the bag. Moreover, such tactile inspection may involve lifting parts of the bag and feeling its weight, which will increase upon reception of faecal matter or urine.

Some human waste management devices, in particular those used as colostomy bags, are found in the marketplace which comprise a transparent plastic material, thus enabling easy visual inspection of the bag.

Such transparent bags offer the advantage of ready inspection in order to establish the need to change the human waste management device. Moreover they provide a means of ready inspection of the consistency of excreted faecal matter or urine. Such bags may be desirable, since the group of typical wearer includes babies or a children and an incontinent adults, all of which may or may not be bedridden. Thus the wearer is typically not only dependent on a caretaker in order to replace the human waste management device, but the caretaker's role will typically also involve a responsibility and an interest in the wearer's health. The consistence of faecal matter or urine excreted by the wearer will give some indication of the wearer's health, namely with regard to his digestive system.

Another advantage associated with transparent bags, is that they allow for easier placement, in particular they allow looking at the aperture of the device from the garment facing side, which can be helpful in placing the device.

Proper placement of a human waste management devices is a problem well known in the art, in particular for those devices designed for adhesive attachment to the wearer's skin. Besides and in connection with optimum adherence, the proper placement of the device is a key issue for the reliable functioning of human waste management devices. Total or substantial misplacement of the device will lead to a severe misfunctioning, in particular incomplete collection of faeces and leaking. If the aperture of the human waste management device is not sufficiently in registry with the anal opening, substantial pressure, in particular on the flanges of the device, can build up in the defecation process. Such substantial pressure can lead to the detachment of the adhesively secured device, obviously entailing the most unwanted consequences.

If the misplacement of the device is recognised before use, the placement of the device is normally corrected, typically by the carer. The necessary detachment and reattachment of the device means an additional stress on the affected areas of skin of the wearer. Many wearers, who make use of human waste management devices have a sensitive skin due to their age, whether very old or very young, and furthermore sometimes also suffer from skin irritations. Proper placement of the device in the first place is therefore highly desirable.

On the other hand, the use of a fully transparent bag is often not desirable. Faecal matter or urine is generally not considered pleasant to look at. Moreover, in some situations people other than the caretaker, may be able to inspect such a transparent bag, for example if no garment is worn, which covers such a human waste management device. Human waste management devices so designed as to be worn under a garment were only recently described in EPO application 97110603.4 ("Shaped fecal management device"), while most human waste management devices disclosed in the prior art are not designed to be worn under a garment. In the case of a human waste management device not covered by a garment ready inspection of the faecal matter or urine contained in the bag of a human waste management device could be considered inappropriate and indignifying, e.g. for a bedridden adult patient and in particular if the faecal matter or urine is of abnormal consistency.

Furthermore, a fully transparent bag is typically provided from a plastic material. Such plastic material is often not desirable for skin contact, since such skin contact gives a sweaty and uncomfortable feeling and may induce additional stress to a wearer's skin. This problem is further increased if the device is worn under clothing.

It has now been found, that all of the above mentioned problems are addressed by a simple and yet effective means disclosed herein.

### Summary of the invention

The present invention relates to a human waste management device (10) comprising a bag (11), said bag (11) having an aperture (21) and a flange (12) surrounding said aperture for adhesive attachment to the uro-genital or perianal area of wearer. The invention resides principally in providing said bag (11) with a transparent area providing a window. In a preferred embodiment of the present invention the bag (11) comprises a window in the garment facing portion (17) of the bag (11) to allow for visual inspection of the inside contents of the bag (11).

### Brief description of the drawings

It is believed that the invention will be better understood from the foregoing description in conjunction with the accompanying drawings in which:
Figure 1 is a perspective view of a preferred embodiment of a faecal management device.
Figure 2 is a perspective view of a diaper and a faecal management device, which can be worn in combination according to the present invention.
Figure 3 is a partially cut-away perspective view of a diaper to be worn in combination with a faecal management device according to the present invention.
Figure 4 is a cut-away side view in the transversal direction of a preferred faecal management device.

### Detailed description of the invention

The invention relates to human waste management devices. Such a human waste management device may be a faecal management device (10), which is designed for attachment to the anal area and mainly used for collecting faeces, or it may be a urinary management device, which is attached to the urinary duct and mainly used for collecting urine. All references to urinary management devices as made herein refer to urinary management devices which comprise a storage means such as a bag, which may also comprise absorbent material. Urinary management devices which do not have any storage capacity and typically serve to close the urethra are not within the scope of the present invention. A human waste management device may also be a device to collect both urine and faeces and thus be attached to both of the above areas. All of the above human waste management devices are preferably designed for single use and disposal thereafter.

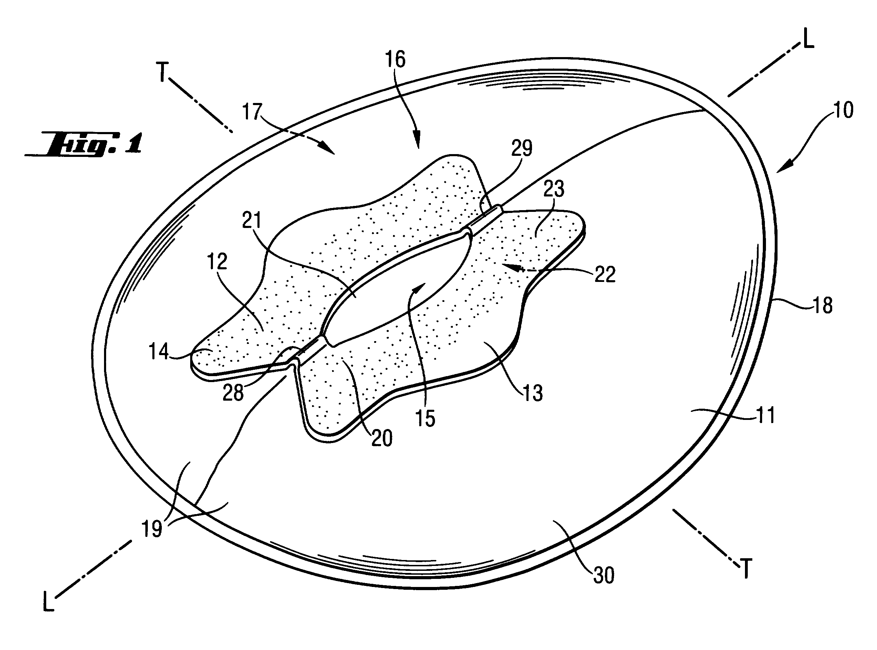

A preferred faecal management device (10) is shown in Figure 1.

### Description of the human waste management device as a whole

Typically human waste management devices comprise a bag (11) having an aperture (21) and a flange (12) surrounding the aperture for preferably adhesive attachment to the perianal area of a wearer as visible from Figure 1. Any human waste management device known in the art can be provided according to the present invention.

The bag (11) as used herein is a flexible receptacle for the containment of excreted faecal matter or urine. The bag (11) is designed to safely contain any entrapped material, typically it will be liquid impermeable, yet it may be breathable. The bag (11) is designed of sufficient strength to withstand rupture in use, also when pressure on the bag (11) is exerted in typical wearing conditions, such as sitting.

According to the present invention the bag material can comprise one or multiple layers, preferably two or three layers. The layer on the inside of the bag (11), which will typically at least partially come in contact with faecal material or urine is called the inner layer. The outermost layer of the bag, which will typically at least partially come in contact with the skin to the wearer and the garments of the wearer, is called the outer layer.

The layers of the bag material may comprise any material, preferably so that the bag is liquid impervious. The preferred optical properties of any such material will be described below in more detail. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon; polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag material may comprise a non-woven layer. Such material layers present an uneven surface to the skin of the wearer and thus reduce significantly the problem of occlusion and greatly improve skin healthiness.

In one preferred embodiment of the present invention the bag material comprises two layers. Preferably the outer layer comprises a non-woven layer and the inner layer comprises a film.

In yet another preferred embodiment of the present invention, the bag material comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the bag material may be hydrophobic or hydrophilic. If the bag material does not comprise a film layer, preferably at least one non-woven layer is hydrophobic. As a consequence, fluid penetration is resisted through the wearer facing portion (16) and the garment facing portion (17) of the human waste management device (10). If the bag material comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, non-anionic, cationic and non-cationic surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

According to the present invention, depending on the shape of the bag (11) required, the bag (11) may be provided from a unitary piece of material or a number of separate pieces of material, which may be identical or different and which are sealed at their respective peripheries. The preferred shape of the bag depends in particular on the intended use thereof, i.e. whether the device is intended for bedridden patients or active patients suffering from incontinence or requiring an artificial bowel or for infants.

The bags described herein preferably have a wearer facing portion (16) and a garment facing portion (17), which both comprise separate pieces of material. The wearer facing portion (16) and the garment facing portion (17) are sealed at the periphery of the bag (11), thus creating a bag peripheral rim (18). The wearer facing portion (16) and the garment facing portion (17) may each independently comprise more than one section of material. Preferably the garment facing portion (17) comprises only one section of material; most preferably also the wearer facing portion (16) comprises only one section of material.

The wearer facing portion (16), the garment facing portion (17) and the pieces of material comprised by either of these portions are secured to each other by means known to the man skilled in the art, such as adhesive, thermobonding or pressure bonding in order to provide the desired bag configuration. The rim (18), at which the wearer facing portion (16) and the garment facing portion (17) are sealed together, may be provided inside the bag (11) rather than outside the bag (11), thus being coextensive with the inner surface (15) of the bag (11) rather than with the outer surface (30) of the bag (11).

Hence a variety of shapes of the bag is within the scope of the present invention. Particularly, preferred shapes are flat circular type bags, cone shaped bags, truncated cone shaped bags and pyramidal or truncated pyramidal shaped bags and flat T shaped bags.

In one embodiment of the present invention the bag (11) may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag or may be secured to the inner surface (15) of the bag (11). Any known techniques for securing absorbent material to nonwoven and film substrates may be used to secure the absorbent material to the inner surface (15) of the bag. The absorbent material may also be arranged to have any desired shape or configuration (e.g., rectangular, oval, circular, etc.).

The bag (11) is provided with an aperture (21) whereby faecal matter or urine is received from the body prior to storage within the bag cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction or in both directions, e.g. the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering faecal matter or urine.

The flange may be provided in any size depending on the wearer group for which the device is intended. Similarly the flange may be provided in any shape and preferably has a symmetrical shape preferably comprising a plurality of lobes (13).

The flange comprises a garment facing portion (22) and a wearer facing portion (23). In an preferred embodiment these are two large, substantially flat surfaces, however, the flange (12) may also comprise projections, a front projection (28) and/or a rear projection (29), in case of a faecal management device (10) designed to fit the perineal and/or coccygeal area of the wearer and in case of a urine management device (10) designed to fit the genital and/or perineal area.

The flange (12) should be made of soft, flexible and malleable material to allow easy placement of the flange (12) to the perianal or uro-genital area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/m², more preferably 50 g/m². Other thermoplastic foam materials, or other suitable plastics sheet materials having the described properties of such foams (i.e., softness, pliability, stretchability, and contractability) might also be used. Preferably, the material of garment facing portion (22) of the flange (12) may extend into the defined aperture area so as to form a skirt or flap of material which prevents unintentional adhesion of the surface edges of the flange (12) defining the aperture (21) to one another during use.

According to the present invention the human waste management device (10) further comprises an attachment means to secure the device to the wearer. Such means include straps and more preferably comprises a body-compatible pressure sensitive adhesive (20) applied to the wearer facing portion (23) of the flange (12).

The adhesive (20) is preferably covered with a release means (not shown) in order to protect the adhesive (20), such as siliconized paper. The adhesive (20) can cover the entire wearer facing portion (23) of the flange (12), more preferably the flange (12) has at least one, preferably two to six non-adhesive portions. These portions may be adhesive free or may contain inactivated or covered adhesives. As is evident from Figure 1, the adhesive is in one preferred embodiment not applied to the entire wearer facing portion (23) of the flange (12), so as to provide lobes (13) on either side of the flange (12) which are non-adhesive and can thereby serve to facilitate placement and removal of the device whilst avoiding contact with the adhesive. These lobes (13) are however preferably also covered by the release means. Before application of the human waste management device (10) to the skin of the wearer, the release means if present is removed.

According to the present invention any medically approved water resistant pressure sensitive adhesive may be used to attach the device to the perianal or uro-genital area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal, are formed from crosslinking polymers with a plastisicer to form a 3-dimensional matrix.

The adhesive (20) can be applied to the wearer facing portion (23) of the flange (12) by any means known in the art such as slot coating) spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example, for human waste management devices (10) to be used for babies the amount of adhesive (20) may be less than for human waste management devices (10) designed for active adult incontinence sufferers.

According to the present invention, depending on the shape of the bag (11) required, the bag (11) may be provided from a unitary piece of material or a number of separate pieces of material, which may be identical or different and which are sealed at their respective peripheries. The preferred shape of the bag depends in particular on the intended use thereof, i.e. whether the device is intended for bedridden patients or active patients suffering from incontinence or requiring an artificial bowel or for infants.

The bags described herein preferably have a wearer facing portion (16) and a garment facing portion (17), which both comprise separate pieces of material. The wearer facing portion (16) and the garment facing portion (17) are sealed at the periphery of the bag (11), thus creating a bag peripheral rim (18). The wearer facing portion (16) and the garment facing portion (17) may each independently comprise more than one section of material. Preferably the garment facing portion (17) comprises only one section of material; most preferably also the wearer facing portion (16) comprises only one section of material.

The wearer facing portion (16), the garment facing portion (17) and the pieces of material comprised by either of these portions are secured to each other by means known to the man skilled in the art, such as adhesive, thermobonding or pressure bonding in order to provide the desired bag configuration. The rim (18), at which the wearer facing portion (16) and the garment facing portion (17) are sealed together, may be provided inside the bag (11) rather than outside the bag (11), thus being coextensive with the inner surface (15) of the bag (11) rather than with the outer surface (30) of the bag (11).

Hence a variety of shapes of the bag is within the scope of the present invention. Particularly, preferred shapes are flat circular type bags, cone shaped bags, truncated cone shaped bags and pyramidal or truncated pyramidal shaped bags. In a most preferred embodiment of the present invention, the bag (11) has a substantially truncated cone shape.

In one embodiment of the present invention the bag (11) may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag or may be secured to the inner surface (15) of the bag (11). Any known techniques for securing absorbent material to nonwoven and film substrates may be used to secure the absorbent material to the inner surface (15) of the bag. The absorbent material may also be arranged to have any desired shape or configuration (e.g., rectangular, oval, circular, etc.).

As shown in Figure 1 the bag (11) is provided with an aperture (21) whereby faecal matter is received from the body prior to storage within the bag cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction or in both directions, e.g. the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering faecal matter.

The flange may be provided in any size depending on the wearer group for which the device is intended. Similarly the flange may be provided in any shape and preferably has a symmetrical shape preferably comprising a plurality of lobes (13)/(14).

The flange comprises a garment facing portion (22) and a wearer facing portion (23). In an preferred embodiment these are two large, substantially flat surfaces, however, the flange (12) may also comprise projections, a front projection (28) and/or a rear projection (29), designed to fit the perineal and/or coccygeal area of the wearer.

The flange (12) should be made of soft, flexible and malleable material to allow easy placement of the flange (12) to the perianal area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/m², more preferably 50 g/m². Other thermoplastic foam materials, or other suitable plastics sheet materials having the described properties of such foams (i.e., softness, pliability, stretchability, and contractability) might also be used. Preferably, the material of garment facing portion (22) of the flange (12) may extend into the defined aperture area so as to form a skirt or flap of material which prevents unintentional adhesion of the surface edges of the flange (12) defining the aperture (21) to one another during use.

According to the present invention the human waste management device (10) further comprises an attachment means to secure the device to the wearer. Such means include straps and more preferably comprises a body-compatible pressure sensitive adhesive (20) applied to the wearer facing portion (23) of the flange (12).

The adhesive (20) is preferably covered with a release means (not shown) in order to protect the adhesive (20), such as siliconized paper. The adhesive (20) can cover the entire wearer facing portion (23) of the flange (12) or more preferably have at least one, preferably two to six non-adhesive portions. These portions may be adhesive free or may contain inactivated or covered adhesives. As is evident from Figure 1, the adhesive is in one preferred embodiment not applied to the entire wearer facing portion (23) of the flange (12), so as to provide lobes (13)/(14) on either side of the flange (12) which are non-adhesive and can thereby serve to facilitate placement and removal of the device whilst avoiding contact with the adhesive. These lobes (13)/(14) are however preferably also covered by the release means. Before application of the human waste management device (10) to the skin of the wearer, the release means if present is removed.

According to the present invention any medically approved water resistant pressure sensitive adhesive may be used to attach the device to the perianal area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal, are formed from crosslinking polymers with a plastisicer to form a 3-dimensional matrix.

The adhesive (20) can be applied to the wearer facing portion (23) of the flange (12) by any means known in the art such as slot coating, spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example, for human waste management devices (10) to be used for babies the amount of adhesive (20) may be less than for human waste management devices (10) designed for active adult incontinence sufferers.

### Detailed description of the invention

To allow a more detailed and clear description of the shape of the bag (11) of the present invention, in the following paragraphs firstly a number of terms, as used herein, will be defined.

The shape of the bag is to understood as the shape exclusively defined by the material forming the bag. While the shape of the bag (11) is thus fully defined by geometrical considerations, the nature of the shape of the bag may be more clearly identified when the bag is inflated with a gas such as air, so that equal pressure is exerted onto the inner layer of the material forming the bag. The pressure should, however, be low enough, so that the material forming the bag is not substantially stretched. Said shape of the bag also determines the volume of the bag.

A section of material is to be understood with regard to the bag (11) as a piece of material cut from the material used for the bag (11), which can be laid flat on a even surface and does not comprise any seals or rims. A section of material may comprise separate layers as described above.

The garment facing portion (17) is the portion of the bag (11), which is generally oriented away from the wearer, when the faecal management device (10) is worn, and towards a garment, if a garment is worn. The garment facing portion (17) does not comprise the aperture (21). The size and the shape of the garment facing portion (17), in particular its length and width, are defined by the bag peripheral rim (18). Sections of material comprised by the rim (18) or seal do not form part of the garment facing portion (17).

The wearer facing portion (16) is the portion of the bag (11), which comprises the aperture (21) and is generally oriented towards the wearer, when the faecal management device (10) is worn. The size and the shape of the wearer facing portion (16) are defined by the bag peripheral rim (18). Sections of material comprised by the rim (18) or seal do not form part of the wearer facing portion (16).

The surface area of the garment facing portion (17), the surface area of the wearer facing portion (16), the surface area of a window (46) are the areas of the outer surfaces (30) of said portions. Material comprised by the rim (18) is not to be taken into account.

Centre as used with reference to the garment facing portion (17) is the centre of the surface area of the garment facing portion (17).

According to the present invention it has been found that a visual inspection means for the human waste management device, such as a window, is essential to the performance and wearer satisfaction as well as the convenience for the caretaker.

Such a visual inspection means may be provided in the form of at least one window (46), which is provided by a transparent area, this transparent area being surrounded by portions of the bag (11) of lower transparency. These portions of lower transparency are referred to as opaque portions. Transparent, as used herein, refers to an opacity (inverse of transparency) of 10 % or less as measured according to ASTM 1003-92. Opaque, as used herein, refers to an opacity of more than 10 % as measured according to ASTM 1003-92.

The windows (46) and other portions of the bag (11) are not identical with regard to their material: The windows (46) are provided from a "window material" whereas the other portions of the bag (11) are provided from a "bag material".

The window material may be selected from any suitable transparent plastic material, among the preferred materials are polyethylene and polyester. The window material may comprise more than one layer and may comprise a laminate of two or more layers. The window material may comprise a non-woven material which provides sufficient transparency, but preferably does not comprise a non-woven material.

Most preferably the window material is provided from a single layer of polyethylene. The caliper of such layer is preferably between 10 micron and 50 micron.

The window material may be joined to the bag material by any means known to the man skilled in the art such as thermobonding, ultrasonic bonding, adhesive means.

The windows (46) may be provided in any shape such as circular, oval, triangular, rectangular, squared. A circular shape is preferred.

The windows (46) may be provided in any size. Preferably, each individual window (46) has a surface area from 1 cm² to 100 cm². Preferably, the total surface area of the windows (46) comprised by the device (10) is from 1 to 99 % of the total surface area of the opaque areas, more preferably from 2 % to 50 %, still more preferably from 5 % to 30 %, most preferably 7 % to 20 %.

The device may comprise any number of windows, preferably one to five windows, more preferably one or two windows. In one preferred embodiment of the present invention a faecal management device for use on a baby is provided, which comprises two windows (46). Both windows (46) are made of transparent polyethylene foil. Both are circular and have a diameter of 5 cm. The garment facing portion (17) of this faecal management device (10) is circular and has a diameter of 18 cm.

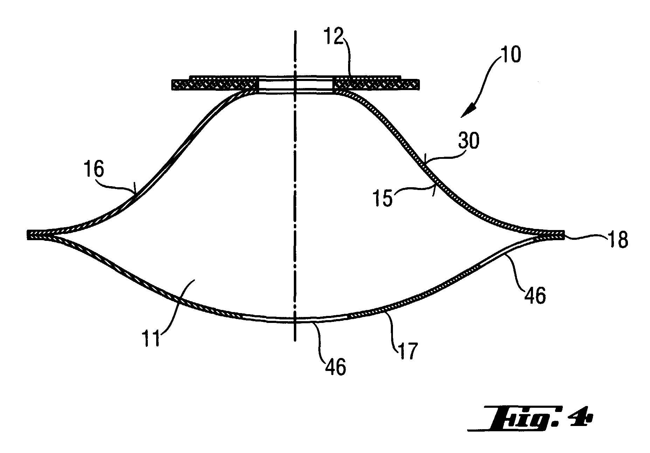

The windows (46) may be provided at any position of the bag (11). In one preferred embodiment the device (10) comprises only one window (46). The window (46) is provided in a central or near central position in the garment facing side of the bag (11). The most preferred position of the window (46) is so that a perpendicular line through the centre of the window (46) crosses the centre of the aperture (21) as indicated in Figure 4.

This position of the window (46) typically allows the person applying the faecal management device (10) to a wearer to see the wearer's anus through the aperture (21). Since for proper functioning of the device (10) the aperture (21) is to be brought in registry with the anus, the window (46) is most useful for the placement of the device (10).

In another preferred embodiment the device (10) comprises only one window (46), which is positioned towards the rear end of the bag (11) either on the wearer facing portion (16) or on the garment facing portion (17). A position on the garment facing portion (17) is preferred. The rear end of the bag (11) is to be understood as the end closest to the coccyx when the faecal management device (10) is worn. For the preferred embodiment of the bag (11), which comprises two sections, a wearer facing portion (16) and a garment facing portion (17), and the bag peripheral rim (18), the window (46) may be provided adjacent to the rim (18) close to the coccygeal end point of the rim (18). The coccygeal end point of the rim (18) is to be understood as the point on the rim (18) which is closest to the wearer's coccyx and thus typically furthest away from the centre of the aperture (21). The rim (18) may or may not comprise portions of the window (46).

In another preferred embodiment the device (10) comprises two windows (46), preferably one in a central position on the garment facing portion (17) and one adjacent to the coccygeal end point of the rim (18).

If a faecal management device (10) is combined with a garment or with a disposable diaper the window (46) is preferably chosen to best suit this combination. The diaper may also be adapted to comprise a window, which is in registry with the window (46) of the human waste management device, so that inspection of the device is possible without removal of the diaper.

In other embodiments of the present invention the bag (11) may be provided with other inspection means, may they be optical or electronic means. Such inspections means may be fully or only in part be comprised by the bag (11) or may be comprised by other parts of the faecal management device (10). One inspection means is a wetness indicator changing its colour due to a chemical reaction.

### Detailed description of a diaper to be worn in combination with the human waste management device

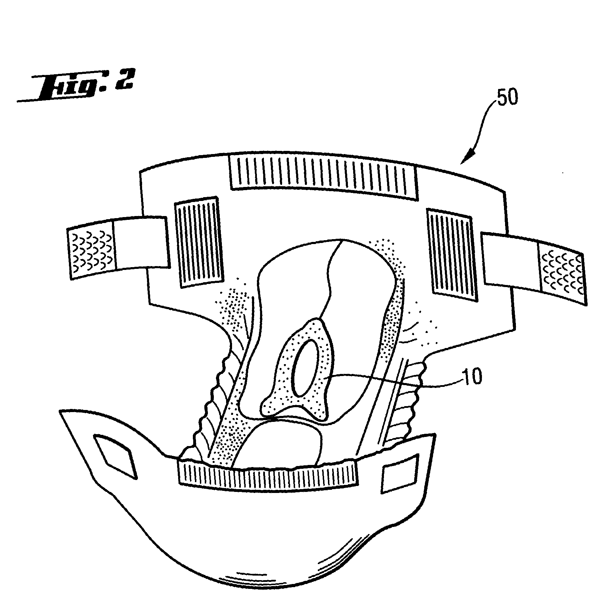

The human waste management device of the present invention has been found to be particularly useful and beneficial when used in conjunction with a garment, or diaper (50), preferably a disposable diaper - refer to Figure 2. In the following paragraphs reference is made to a faecal management device (10) while a urine management device is as suitable for the combination with a diaper (50). For example a faecal management device (10) is preferably first placed in the perianal area of the wearer before the disposable diaper (50) is applied. In particular, the diaper (50) is positioned over the faecal management device (10) and fastened in a conventional manner around the body of the wearer. It has been found that, in addition, to providing excellent separation between urine and faecal material, the combined faecal management device (10) and diaper (50) system actually reduces skin irritation, which may at times occur, especially since the group of typical wearers includes the very old, the very young and the unhealthy wearers. In effect, the presence of the faecal management device (10) permits the formation of a separation layer between the skin of the wearer and the diaper (50), i. e. a part of the absorbent core (58) of the diaper (50). The diaper (50) can be of the conventional type (an embodiment of which is described below although not a limiting example by any means) or can be adapted to contain in an effective and comfortable manner the faecal management device (10) according to the teachings of the present invention.

As used herein, the term "disposable diapers" refers to articles which absorb and contain body extrudates; and more specifically, refers to articles which are placed against or in proximity to the body of the wearer to absorb and contain the various extrudates discharged from the body and which are intended to be discarded after a single use (i. e., they are not intended to be laundered or otherwise restored or reused) and, preferably, to be recycled, composted or otherwise disposed of in an environmentally compatible manner. As used herein, the term "diaper" refers to a garment generally worn by infants or incontinence sufferers that is drawn up between the legs and fastened about the waist of the wearer.

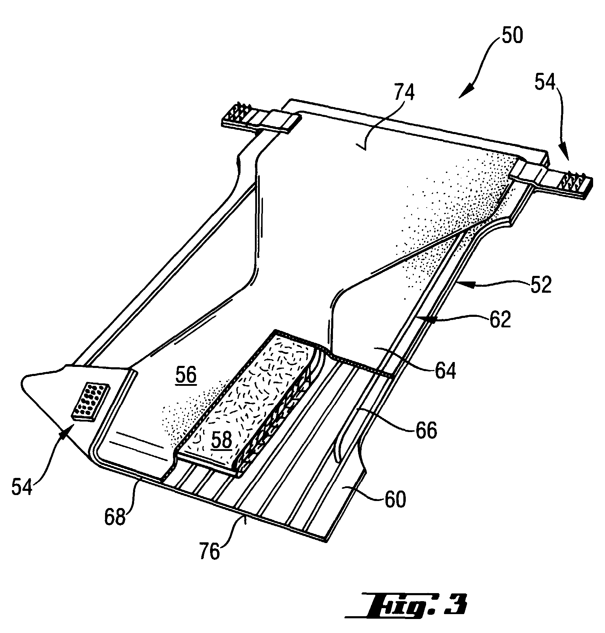

Figure 3 is a partially cut-away perspective view of a diaper (50) embodying the present invention prior to it being placed on the wearer over the faecal management device (10). As is visible from Figure 3, a preferred diaper (50) comprises a body portion (52) and a refastenable mechanical fastening device (54). A preferred body portion (52) comprises a liquid pervious topsheet (56), and absorbent core (58), a liquid impervious backsheet (60), and elastically contractible leg cuffs (62); each leg cuff (62) preferably comprising a side flap (64) and one or more elastic members (66). For simplicity purposes, only one elastic member (66) is shown in the side flap (64). While the topsheet (56), the absorbent core (58), the backsheet (60), the side flaps (64), and the elastic members (66) may be assembled in a variety of well-known configurations. A preferred disposable diaper configuration is shown and generally described in US 3,860,003, an even more preferred disposable diaper configuration is shown and generally described in WO 93/16669. In this preferred diaper configuration, the backsheet (60) is joined to the topsheet (56); the absorbent core (58) is positioned between the topsheet (56) and the backsheet (60); the side flaps (64) extend outwardly from and along each side edge of the absorbent core (58); and the elastic member (66) is operatively associated with each side flap (64).

Figure 3 shows the body portion (52) in which the topsheet (56) and the backsheet (60) are coextensive and have length and width dimensions generally larger than those of the absorbent core (58). The topsheet (56) is superposed on the backsheet (60) thereby forming the periphery (68) of the body portion (52).

The body portion (52) has an inside surface (74) and an outside surface (76). When a backsheet (60) is used, it typically forms the outside surface (76) of the body portion (52). The inside surface (74) is that surface of the diaper (50) opposite the outside surface (76) and in the embodiment shown is typically formed by the topsheet (56). In general, the inside surface (74) of the diaper (50) is that surface coextensive with the outside surface (76) and which is for the greater part in contact with the wearer when the diaper (50) is worn.

The absorbent core (58) of the body portion (52) may be any absorbent means which is generally compressible, conformable, non-irritating to the skin of the wearer, and capable of absorbing and retaining liquids such as urine and other certain bodily discharges. The absorbent core (58) may be manufactured in a variety of sizes and shapes (for example, rectangular, hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, meltblown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core (58) may also be varied (for example, the absorbent core (58) may have varying caliper zones, hydrophilic gradients, superabsorbent gradients, or lower average density and lower average basis weight acquisition zones; or may comprise one or more layers or structures). Further, the size and absorbent capacity of the absorbent core (58) may be varied to accommodate wearers ranging from infants to adults.

The backsheet (60) is impervious to liquids (for example, urine) and is preferably manufactured from a thin plastic film, preferably a thermoplastic film, although other flexible liquid impervious materials may also be used. As used herein, the term "flexible" refers to materials which are compliant and which will readily conform to the general shape and contours of the human body. The backsheet (60) prevents the exudates absorbed and contained in the absorbent core (58) from soiling articles which are in contact with the diaper (50) such as undergarments and bedding. The backsheet (60) may thus comprise polymeric films such as thermoplastic films of polyethylene or polypropylene, or composite materials such as film-coated non-woven material. Exemplary films are manufactured by Tredegar Industries, Inc. of Terre Haute, Ind., USA or BP-Chemical PlasTec, Rotbuchenstrasse 1, D-8000 München, Germany.

The backsheet (60) is preferably textured to provide a more clothlike appearance. Further, the backsheet (60) may also permit vapours to escape from the absorbent core (58) while still preventing exudates from passing through the backsheet (60) by, for example, being supplied with microapertures. The size of the backsheet (60) is dictated by the size of the absorbent core (58) and the exact diaper design selected.

The topsheet (56) of the diaper is compliant, soft feeling and non-irritating to the skin of the wearer. Further, the topsheet (56) is liquid pervious permitting liquids (for example, urine) to readily penetrate through its thickness. A suitable topsheet (56) may be manufactured from a wide range of materials, such as porous foams, reticulated foams, apertured films; or woven or non-woven webs of natural fibres (for example, wood or cotton fibres) or from a combination of natural and synthetic fibres. Preferably, it is made of a material that isolates the skin of the wearer from liquids retained in the absorbent core (58).

There are a number of manufacturing techniques which may be used to manufacture the topsheet (56). For example, the topsheet (56) may be a non-woven web of fibres. An exemplary topsheet (56) is carded and thermally bonded by means well-known to those skilled in the fabric art. A suitable topsheet (56) is manufactured by, for example, Veratec Inc., a division of International Paper Company, of Walpole, Mass., USA. A topsheet (56) particularly preferred for incontinence garments comprises a formed thermoplastic film.

## Claims

1. Human waste management device comprising a bag (11), characterised in that said bag (11) comprises at least one transparent area and at least one opaque area.

2. Human waste management device according to Claim 1, characterised in that said transparent areas have a total surface area of 7 % to 20 % of the total surface area of said opaque areas.

3. Human waste management device according to any one of the preceding claims, characterised in that said transparent areas each independently have a surface area between 1 cm² and 100 cm².

4. Human waste management device according to any one of the preceding claims, characterised in that said bag (11) comprises only one transparent area.

5. Human waste management device according to any one of the preceding claims, characterised in that said transparent areas substantially consist of a plastic material.

6. Human waste management device according to any one of the preceding claims, characterised in that said opaque areas comprise a non-woven material.

7. Human waste management device according to Claim 6, characterised in that said opaque areas comprise a laminate comprising said non-woven material.

8. Human waste management device according to any one of the preceding claims, characterised in that said bag (11) comprises a wearer facing portion (16), a garment facing portion (17) and a bag peripheral rim (18).

9. Human waste management device according to Claim 8, characterised in that at least one of said transparent areas comprises the centre of said garment facing portion (17).

10. Human waste management device according to Claim 8, characterised in that at least one of said transparent areas is adjacent to the coccygeal end point of said bag peripheral rim (18).
